# EUROPÄISCHE PATENTANMELDUNG

(11) **EP 0 706 996 A2**
(43) Veröffentlichungstag der Anmeldung: **17.04.1996**
(21) Anmeldenummer: 95111037.8
(22) Anmeldetag: 14.07.1995
(51) Int. Cl.: C07C 275/14, C08G 59/54

(54) **Harnstoffgruppen enthaltende Polyamine**

(30) Priorität: 14.09.1994 DE 4432637
(71) Anmelder: HÜLS AKTIENGESELLSCHAFT, D-45764 Marl (DE)
(72) Erfinder: Gras, Rainer, Dr., D-44879 Bochum (DE); Wolf, Elmar, Dr., D-45661 Recklinghausen (DE)

(57) **Zusammenfassung**

Gegenstand der vorliegenden Erfindung sind neue Harnstoffgruppen enthaltende Polyamine sowie ein Verfahren zur Herstellung dieser Polyamine.

Harnstoffgruppen enthaltende Polyamine der folgender Zusammensetzung: 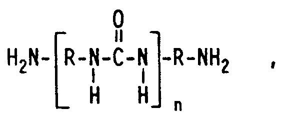
wobei 2 ≦ n ≧ 1 und R ein Alkylen-Rest mit 2 - 14 C-Atomen, der gegebenenfalls mit 1 - 3 CH₃- oder C₂H₅-Gruppen substituiert sein kann, bedeutet, und 1 - 3 CH₂-Gruppen des Alkylenrestes R durch -O-, -NH-, -NCH₃-substituiert sein können.

## Beschreibung

Gegenstand der vorliegenden Erfindung sind neue Harnstoffgruppen enthaltende Polyamine sowie ein Verfahren zur Herstellung dieser Polyamine.

Die mit Polyaminen gehärteten Epoxid-Harze auf Bisphenol A-Basis zeichnen sich in der Praxis durch eine Reihe von erwünschten Eigenschaften aus, wie z. B. gute Haftung auf allen möglichen Substraten, gute Lösungsmittelbestsändigkeit und Resistenz gegenüber Chemikalieneinwirkung. Diese Eigenschaften der gehärteten Polyamin/Epoxid-Gemische könnten noch verbessert werden, wenn die Polyamine zusätzlich Harnstoffgruppen enthielten. Die Herstellung Harnstoffgruppen enthaltender Polyamine durch Kondensation von 2 mol Polyamin und 1 mol Harnstoff führt zu festen bis hochviskosen Produkten, die lösemittelfrei nicht verarbeitbar sind.

Aufgabe der vorliegenden Erfindung war es somit, ein Verfahren aufzuzeigen, das die Herstellung von Harnstoffgruppen enthaltenden Polyaminen, die bei Raumtemperatur mit Epoxid-Harzen verarbeitbar sind, ermöglicht.

Überraschenderweise konnte diese Aufgabe dadurch gelöst werden, daß Harnstoff mit dem Polyamin kondensiert wird, wobei die Polyaminkomponente im großen Überschuß vorliegt, und das nicht umgesetzte Polyamin durch Dünnschichtverdampfung vom Reaktionsprodukt, dem Harnstoffgruppen enthaltenden Polyamin, entfernt wird.

Gegenstand der vorliegenden Erfindung sind somit harnstoffgruppenhaltige Polyamine folgender Zusammensetzung: 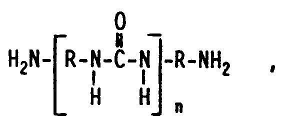
wobei 2 ≦ n ≧ 1 und R ein Alkylen-Rest mit 2 - 14 C-Atomen, der gegebenenfalls mit 1 - 3 CH₃- bzw. C₂H₅-Gruppen substituiert sein kann, bedeutet, und 1 - 3 CH₂-Gruppen des Alkylenrestes R durch -O-, -NH-, -NCH₃-substituiert sein können.

Die erfindungsgemäßen Verbindungen sind gekennzeichnet durch einen basischen Amingehalt von 4 - 14 mmol NH₂/g.

Die Konzentration an Harnstoffgruppen bei den erfindungsgemäßen Polyaminen beträgt 4 - 13 mmol/g. Die Viskosität bei 25 °C der erfindungsgemäßen Verbindungen variiert von 5 000 - 100 000 mPas.

Gegenstand der vorliegenden Erfindung ist außerdem ein Verfahren zur Herstellung von bei Raumtemperatur verarbeitbaren Harnstoffgruppen enthaltender Polyamine, dadurch gekennzeichnet, daß man Harnstoff mit Polyaminen folgender Zusammensetzung:

H₂N-R-NH₂,

wobei R ein Alkylenrest mit 2 - 14 C-Atomen, der gegebenenfalls mit 1 - 3 CH₃- oder C₂H₅-Gruppen substituiert sein kann, bedeutet, und 1 - 3 CH₂-Gruppen des Alkylenrestes R durch -O-, -NH-, NCH₃-Gruppen substituiert sein können, im Molverhältnis Harnstoff zu Polyamin gleich 1:(5-20) so umsetzt, daß zunächst bei 130 - 150 °C solange erhitzt wird, bis ca. 80 % der berechneten NH₃-Menge freigesetzt worden sind, danach langsam auf 200 °C bis zur quantitativen NH₃-Abspaltung weiter erhitzt wird, und nach erfolgter Umsetzung das nicht umgesetzte Polyamin durch Dünnschichtverdampfung bei 100 - 180 °C und 0.1 Torr vom Reaktionsprodukt abgetrennt wird.

Bei den im Sinne der vorliegenden Erfindung einzusetzenden Polyaminen handelt es sich um offenkettige Amine, wie z. B. Ethylendiamin, 1.2-Propandiamin, 1.3-Diaminopropan, 1.4-Diaminobutan, Neopentandiamin, 2-Methylpentamethylendianin, 5-Methylnonamethylendiamin, 4.7-Dioxadecan-1.10-diamin, 4.9-Dioxadodecan-1.12-diamin, 4.7.10-Trioxatridecan-1.13-diamin, 2.2.4(2.4.4)-Trimethylhexamethylendiamin, Diethylentriamin, Dipropylentriamin, N.N'-Bis-(3-aminopropyl)-ethylendiamin.

Gemäß dem erfindungsgemäßen Verfahren werden Harnstoff und die angegebenen Polyamine im Molverhältnis 1:(5-20) miteinander umgesetzt, und nach erfolgter Umsetzung, d. h. NH₃-Abspaltung, wird in einer 2. Stufe das nicht umgesetzte Polyamin durch Dünnschichtverdampfung abgetrennt.

Bei der Kondensation des Harnstoffs mit dem Polyamin werden die Komponenten im angegebenen Molverhältnis bei 130 - 150 °C so lange erhitzt, bis ca. 80 % der zu erwartenden NH₃-Menge freigesetzt worden sind. Anschließend wird zur Vervollständigung der Reaktion noch langsam auf 200 °C erhitzt und so lange weiter erhitzt, bis die NH₃-Abspaltung quantitativ erfolgt ist. Danach wird das Reaktionsgemisch abgekühlt und nach einer Zwischenlagerung bei Raumtemperatur das nicht umgesetzte Polyamin bei 100 - 180 °C und 0.1 Torr mittels Dünnschichtverdampfung entfernt. Die Temperatur, bei der die Abtrennung des Polyamins durch Dünnschichtverdampfung erfolgt, hängt vom Siedepunkt des abzutrennenden Polyamins ab. Je hoher dessen Siedepunkt, desto höher liegt auch die Temperatur, bei der die Abtrennung des Polyamins im Dünnschichtverdampfer (bei einem Vakuum von ca. 0.1 Torr) erfolgt.

Die erfindungsgemäßen Verbindungen eignen sich in hervorragender Weise zur Härtung von Epoxid-Harzen bei Raumtemperatur.

### Allgemeine Herstellungsvorschrift für die Kondensation von Polyaminen mit Harnstoff

Harnstoff und das Polyamin werden im Molverhältnis 1:(5-20) bei ca. 150 °C unter intensiver Rührung so lange erhitzt (ca. 5 h), bis ca. 80 % der NH₃-Menge freigesetzt worden sind. Anschließend wird zur Vervollständigung der Reaktion auf 200 °C erhitzt und ca. 1 h bei dieser Temperatur weiter erhitzt. In der Regel ist die Reaktion unter diesen Bedingungen beendet.

Der bei der Reaktion entweichende Ammoniak wird in einer 2 n H₂SO₄-Lösung aufgefangen, deren Gehalt in regelmäßigen Abständen geprüft wird. Auf diese Weise läßt sich die Reaktion leicht verfolgen.

### Beispiel 1

a) Harnstoff wurde mit 2-Methylpentamethylendiamin im Molverhältnis 1:8 unter den in der allgemeinen Herstellungsvorschrift für die Kondensation von Polyaminen mit Harnstoff beschriebenen Bedingungen zur Reaktion gebracht. Das Reaktionsgemisch hatte einen bas. Amingehalt von 14.3 mmol NH₂/g.
b) Zur Abtrennung des nicht umgesetzten 2-Methylpentamethylendiamins wurde das Reaktionsgemisch 1a bei 100 °C und 0.1 mbar gedünnschichtet. Das Reaktionsprodukt, der Rückstand der Dünnschichtverdampfung, hatte eine Viskosität (bei 25 °C) von 12 500 mPas; der basische Amingehalt betrug 6.8 mmol NH₂/g; der 2-Methylpentamethylendiamin-Gehalt war < 0.7 %.

### Beispiel 2

a) Harnstoff wurde mit 2.2.4(2.4.4)-Trimethylhexamethylendiamin (TMD) im Molverhältnis 1:10, wie im Beispiel 1a beschrieben, umgesetzt. Das Reaktionsgemisch hatte einen basischen Amingehalt von 10.9 mmol NH₂/g.
b) Zur Abtrennung des nicht umgesetzten TMD wurde das Reaktionsgemisch 2a bei 110 °C und 0.1 mbar gedünnschichtet. Das Reaktionsprodukt hatte eine Viskosität (bei 25 °C) von 70 000 mPas; der basische Amingehalt betrug 5.1 mmol NH₂/g; der TMD-Gehalt betrug 0.6 %.

### Beispiel 3

a) Harnstoff wurde mit Diethylentriamin im Molverhältnis 1 : 10, wie im Beispiel 1a beschrieben, umgesetzt. Das Reaktionsgemisch hatte einen basischen Amingehalt von 16.8 mmol NH₂/g.
b) Zur Abtrennung des nicht umgesetzten Diethylentriamins wurde das Reaktionsgemisch 3a bei 100 °C und 0.1 mbar gedünnschichtet. Das Reaktionsprodukt hatte eine Viskosität (bei 25 °C) von 15 200 mPas; der basische Amingehalt betrug 11.5 mmol NH₂/g; der Diethylentriamingehalt war < 0.6 %.

### Beispiel 4

a) Harnstoff wurde mit 5-Methylnonamethylendiamin im Molverhältnis 1:15, wie im Beispiel 1a beschrieben, umgesetzt. Das Reaktionsgemisch hatte einen basischen Amingehalt von 10.6 mmol NH₂/g.
b) Zur Abtrennung des nicht umgesetzten 5-Methylnonamethylendiamins wurde das Reaktionsgemisch 4a bei 110 °C und 0.1 mbar gedünnschichtet. Das Reaktionsprodukt hatte eine Viskosität (bei 25 °C) von 51 000 mPas; der basische Amingehalt betrug 5.1 mmol NH₂/g; der 5-Methylnonamethylendiamingehalt war < 0.7 %.

## Patentansprüche

1. Harnstoffgruppen enthaltende Polyamine der folgender Zusammensetzung: 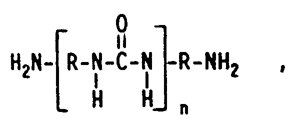 wobei 2 ≦ n ≧ 1 und R ein Alkylen-Rest mit 2 - 14 C-Atomen, der gegebenenfalls mit 1 - 3 CH₃- oder C₂H₅-Gruppen substituiert sein kann, bedeutet, und 1 - 3 CH₂-Gruppen des Alkylenrestes R durch -O-, -NH-, -NCH₃- substituiert sein können.

2. Verfahren zur Herstellung von bei Raumtemperatur verarbeitbarer Harnstoffgruppen enthaltender Polyamine, dadurch gekennzeichnet, daß man Harnstoff mit Polyaminen folgender Zusammensetzung
H₂N-R-NH₂,
wobei R ein Alkylenrest mit 2 - 14 C-Atomen, der gegebenenfalls mit 1 - 3 CH₃- oder C₂H₅-Gruppen substituiert sein kann, bedeutet, und 1 - 3 CH₂-Gruppen des Alkylenrestes R durch -O-, -NH-, -NCH₃-Gruppen substituiert sein können, im Molverhältnis Harnstoff zu Polyamin gleich 1:(5-20) so umsetzt, daß zunächst bei 130 - 150 °C solange erhitzt wird, bis ca. 80 % der berechneten NH₃-Menge freigesetzt worden sind, danach langsam auf 200 °C bis zur quantitativen NH₃-Abspaltung weiter erhitzt wird, und nach erfolgter Umsetzung das nicht umgesetzte Polyamin durch Dünnschichtverdampfung bei 100 - 180 °C und 0.1 Torr vom Reaktionsprodukt abgetrennt wird.

3. Verwendung der Harnstoffgruppen enthaltenen Polyamine nach Anspruch 1 zur Härtung von Epoxid-Harzen bei Raumtemperatur.
